# EUROPEAN PATENT SPECIFICATION

(11) **EP 2 932 972 B1**
(45) Date of publication and mention of the grant of the patent: **18.07.2018**
(21) Application number: 13862018.2
(22) Date of filing: 11.12.2013
(51) Int. Cl.: A61K 31/7048, A61P 5/16, A61P 9/00, A23L 29/00

(54) **NEW APPLICATION OF CATALPOL**
NEUE VERWENDUNG VON CATALPOL
NOUVELLE APPLICATION DE CATALPOL

(30) Priority: 12.12.2012 CN 201210538830
(43) Date of publication of application: 21.10.2015
(73) Proprietor: SUZHOU YOUSEEN NEW PHARMACY DEVELOPMENT CO., LTD., Suzhou Industrial Park Suzhou Jiangsu 215028 (CN)
(72) Inventor: XUAN, Zhenyu, Pudong Shanghai 201203 (CN); HUA, Yan, Shanghai 201203 (CN); CHEN, Changxun, Shanghai 201203 (CN); LIU, Danfeng, Shanghai 201203 (CN); ZHOU, Chi, Shanghai 201203 (CN)
(74) Representative: Brown, David Leslie
(86) International application number: PCT/CN2013/089046
(87) International publication number: WO 2014/090151

(56) References cited:
- CN-A- 102 008 497
- Florian Ploberger: "Das TCM-Rezeptierbuch", 2007, Elsevier, Urban & Fischer, München, Jena, XP002758577, ISBN: 978-3-437-57840-3 pages 107-109, * the whole document * * Yin -Mangel and Liu wei di huang wan (6-Bestandteile-Pille mit Rx. Rehmanniae; page 107 * * Entsprechende westliche Krankheiten Hyperthyreose; page 108 *
- LIU QINGFENG ET AL.: 'Regulatory action of catalpol from Radix Rehmanniae on ?-adrenoceptor-cyclic AMP system.' JOURNAL OF RADIOIMMUNOLOGY. vol. 17, no. 5, 2004, pages 358 - 360, XP055256086
- LIU QINGFENG ET AL.: 'Dual direction regulatory effect of catalpol on imbalance of ?-adrenoceptor and M-cholinergic receptor.' JOURNAL OF SHANGHAI JIAOTONG UNIVERSITY (MEDICAL ( SCIENCE ). vol. 27, no. 12, December 2007, pages 1432 - 1434, XP008179324

## Description

### Field of Invention

The present invention relates to a novel usage of Catalpol, and in particular, relates to an application thereof in the preparation of drugs or health food for treatment of hyperthyroidism.

### Background Art

Hyperthyroidism, also named 'hyperthyrea', is a clinical syndrome of excess of thyroid hormone entering blood circulation and acting on body tissues and organs, due to various diseases, and exemplified by hypermetabolism and increased neural excitation. Its incidence increases significantly with the increase of societal and work pressure and heightened pace of life. Epidemic research indicates that male-female incidence is 1:4-1:6, with patients mostly of 20-40 years age, and particularly female ones. In China, overall incidence is as high as 3%.

Clinical manifestations of hyperthyroidism, with varied degrees of severity in symptoms, include: ①nervous system: excitability, nervous irritability, logorrhea, hyperkinetic syndrome, insomnia, nervousness, attention dysfunction, anxiety, dysphoria, and paranoia; a patient manifests slight tremor when he shows his tongue or extends his both arms forward horizontally; ②hypermetabolism syndrome: the patient has increased perspiration, is heat intolerant, has frequent low fever, has warm and damp skin, and is fatigue and weakness prone; ③thyroid enlargement: the enlargement is mostly diffuse and symmetric, and asymmetric in few cases; it occurs mostly with young females; in a physical examination, contractive noise of the thyroid is audible, and a tremor is perceivable, and is particularly apparent with the upper part of the thyroid, which is a characteristic bodily feature; ④eye features: eye balls of a patient bulge, and the eyeslit breadth increases; the eyelid retraction, as is so called, is differentiated as invasive and non-invasive; ⑤cardiovascular system: a patient manifests cardiopalmus and short breath, and particularly so when engaged in an activity; also manifested are tachycardia (mostly sinus type), arrhythmia, cardiac hypertrophy, and congestive heart failure; ⑥digestive system: appetite increases substantially while weight decreases, with defection frequency increase observed in some cases; ⑦motion system: muscle asthenia; ⑧reproductive system: female patients manifest decrease in menstrual amount, prolonged menstrual cycle, and even amenorrhea, while male ones manifest impotence; ⑨skin and extremity: a few patients manifest symmetric myxedema, mostly on the lower anterior tibia; ⑩ coma and peril to life, in cases of severity.

Cause and mechanism of hyperthyroidism is unknown, with western medicine attributing the cause of the disease to excess of thyroid secretion, which is influenced by the thyroid stimulating hormone (TSH) of the anterior pituitary. At occurrence of nervous disorders, the anterior pituitary secrets excessive thyroid stimulating hormone and elicits thyroid hyperplasia or hypertrophy. While the excess of thyroid stimulating hormone increases metabolism rate, speeding up oxidation process, resulting in various hyperthyroidism syndromes, and inducing metabolism disorders in energy, sugar, fat, protein and vitamins and function disorders in circulatory, digestive, hematopoietic, nervous and endocrine systems. Other theories attribute the leading role of the disease to hereditary and auto-immune factors.

Currently, drugs for the disease, though various, are defective or have side effects in one way or another. In China, common therapy for the disease is drug medication using anti thyroid drugs (ATD), β blockers, iodine preparations, and glucocorticoid, and so on. The most common ATDs include methimazole (MMI) and propylthiouracil (PTU), with their major mechanism being inhibition of synthesis of thyroid stimulating hormone. β blockers may be used to tackle adrenergic effects of a patient with symptoms and signs of increased adrenergic activity, thereby mitigating tachycardia, nervousness, increased perspiration, tremor and myopathy. Propranolol hydrochloride, the commonly used β blocker, can also reduce 5'-deiodinase activity, thus inhibiting transfer from T4 to T3. Iodide is the most ancient anti-thyroid drug. It reduces blood supply to thyroid glands, thus reducing size of the latter, inhibiting synthesis and release of the thyroid stimulating hormone, and therefore has notable and quick therapeutic effects. However, an 'escape' phenomenon may occur after 2-3 weeks of usage, thus aggravating the disease and complicating follow-up therapies. In clinical practice, compound iodine solution is used in hyperthyroidism crisis, preoperative preparation, and as a radioiodine drug. Currently recognized glucocorticoid is mainly used for therapy of thyroid associated ophthalmopathy (TAO), hyperthyroidism crisis, and severe subacute thyroiditis. At the mean time, adverse reaction of the ATD has attracted attention of scholars. In a word, ATD is therapeutically safe and effective, albeit adverse reaction is common, such as toxic effect on the liver and the blood system, antineutrophilic cytoplasmic antibody associated pulmonary small vessel vasculitis, hypoglycemia, allergy, and muscle injury. The adverse reaction is generally slight, self recovery is possible if timely ATD drug withdrawal is exercised. However, in some rare cases, severe adverse reaction might take place to the extent of life threatening, and thus warranting attention from clinicians. MMI, as compared with PTU, has less adverse effects, with the former dosage dependent, while the latter not significantly correlated with dosage. In addition, PTU is more liver toxic than MMI, and might cause life threatening liver injury or exhaustion. Thiourea ATDs have side effects of drug eruption, agranulocytosis, severe liver injury, antineutrophilic cytoplasmic antibody (ANCA) associated vessel vasculitis, in addition to being teratogenic to certain extent. Severe liver injury and ANCA associated vessel vasculitis is frequently related with propylthiouracil, while teratogenic effects are more often seen with methimazole. Treatment with traditional Chinese medicine employs compound decoctions, is generally case-based, lacking clinical statistics.

Catalpol is an iridoids monoglycoside compound, with molecular formula being C₁₅H₂₂O₁₀. It is extremely resourceful and widely distributed among sympetalae plants in genera of Buddleia, Loganiaceae; Paulownia Sieb. Et Zucc, Scrophulariaceae; Catalpa, Bignoniaceae; Cistanche, Orobanchaceae, and Plantago, Plantaginaceae.

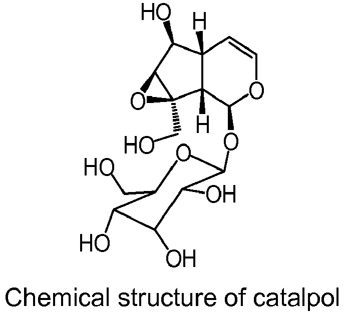

### Summary of the Invention

The object of the present invention is to provide a novel usage of catalpol, as a drug for clinical application.

For realization of the afore-mentioned object, the present invention adopts the following technical solution:
A novel usage of catalpol, that is, the catalpol is employed in the preparation of a drug or health food for treatment of hyperthyroidism.

A novel usage of catalpol, that is, the catalpol is employed in the preparation of a drug or health food for treatment of hyperthyroidism related cardiopathy. Liu Qingfeng et al.:Regulatory action of catalpol from Radix Rehmanniae on beta-adrenoceptor-cyclic AMP system". Journal of Radioimmunology, 2004,vol.17,no. 5,pp.358-360.

Liu Qingfeng et al.:"Dual direction regulatory effect of catalpol on imbalance of beta-adrenoceptor and M-cholinergic receptor". Journal of Shanghai Jiaotong University (Medical Science), December 2007,vol. 27,no.:12, pp.1432-1434. Florian Ploberger: "Das TCM-Rezeptierbuch", 2007, Elsevier, Urban & Fischer, München,Jena, XP002758577, ISBN: 978-3-437-57840-3, pp. 107-109.

### Embodiments

The present invention will be expounded in a detailed and integral manner in combination with the ensuing embodiments.

### 1 Material

### 1.1 Experimental animal

Kunming mice in CL grade, 18-25 grams.

### 1.2 Drugs

Test drug: catalpol (20 grams).

Control drugs: levothyroxine sodium and methimazole.

### 1.3 Reagents

Iodine [¹²⁵I] triiodothyronine (T₃) radioimmunoassay kit; Iodine [¹²⁵I] thyroxine (T₄) radioimmunoassay kit; Iodine [¹²⁵I] free triiodothyronine (FT₃) radioimmunoassay kit; Iodine [¹²⁵I] free thyroxine (FT₄) radioimmunoassay kit.

### 1.4 Instruments

JA5003A electronic balance; ECG-6511 electrocardiograph; model CY-3 digital oxygen meter; SN-697 immunoassay γ counter; KK25E73TI Siemens refrigerator; Scanspeed mini centrifuge; DL-50RC hypothermic centrifuge.

### 2 Methods

### 2.1 Animal model preparation

The animals are placed indoor for milieu adaptation 2 days prior to the experiment. 20 Kunming mice are randomly selected and put in the blank group, administered normal saline by subcutaneous injection, while the other mice are administered levothyroxine sodium 350µg/kg by subcutaneous injection, with a daily dosage of 10ml/kg, for 7 consecutive days.

After the third hour at the sixth day of medication, 10 mice (half male and half female) are randomly selected respectively from the blank group and the model group for measurement of heart rate. Under anesthesia, the mice are taken electrocardiogram for calculation of their heart rate. Statistical analysis is then conducted for comparison of differences.

In the case of substantial acceleration of its heart rate, a mouse is suspended of food without suspension of water for 12 hours starting at 8 o'clock of the last day (the seventh day) of medication. Each mouse in each group is taken of its weight at 8 o'clock the next morning. Blood is taken from its eye ball and subsequent to coagulation, put in centrifugation for 15 minutes at 3500 r/min, for measurement of T₃ and T₄ by means of radioimmunoassay. Each mouse is sacrificed and its heart is taken out for measurement of its heart weight and left ventricular weight. At conclusion of the experiment, the heart weight index and left ventricular weight index is calculated. [Heart weight index HWI=heart weight (HW)/body weight (BW); left ventricular weight index LVWI= left ventricular weight (LVW)/ body weight (BW)]

If the levels of the above indexes are substantially elevated, the model is regarded as successful.

### 2.2 Animal Allocation and Treatment

The successfully modeled mice are randomized into 5 groups (10 mice per group), that is, the hyperthyroidism model group, the catalpol low dosage group, the catalpol medium dosage group, the catalpol high dosage group, and the methimazole group, plus the blank group (10 mice).

The catalpol low dosage group is administered 30mg/kg catalpol, the catalpol medium dosage group 60mg/kg, the catalpol high dosage group 90mg/kg and the methimazole group 7.5mg/kg methimazole, all by gastric infusion, while the blank group and the hyperthyroidism group are administered equal amount of distilled water, once per day, for 20 consecutive days. With the exception of the blank group, each mouse in each group continues to be administered 350µg/kg levothyroxine sodium by means of subcutaneous injection each day.

### 2.3 Index measurement and methods

### 2.3.1 Observation of appearance and behavior of the mice

Animals were observed and recorded daily for their general status, including mental status, color and hair change, appearance and behavior. In addition, every two days, record is taken of their food intake.

### 2.3.2 Measurement of mice weight change

A mouse is suspended of food without suspension of water for 12 hours starting at 8 o'clock one day prior to the first medication, and all the mice are taken of their body weight (BW) the next day, that is, the pre-treatment body weight (BW1); the mouse is suspended of food without suspension of water for 12 hours starting at 8 o'clock of the day of the last medication, its body weight (BW) is taken the next day, that is, the post-treatment body weight (BW2); the difference of the pre-treatment body weight and the post-treatment body weight is calculated to compare the change of the body weight of the mice among each group.

### 2.3.3 Measurement of the oxygen consumption of the mice

Oxygen consumption is measured two days prior to the last medication. The specific method is: take a model CY-3 digital oxygen meter and install therein an oxygen electrode. Connect, perform zero adjustment, and repeat until the instrument is stable for use. Take a 500ml bottle, fix its outward connecting pipe with a rubber cover, seal with Vaseline. The rubber cover is connected via a tee, and a shift in the tee is selectable for connection or disconnection with outer space. A mouse is placed inside the bottle, and the mouth of the bottle is instantly covered with a wood plug to shut off air from the inner flask. The filtering flask is horizontally placed on the experiment table, and timing of 20 minutes After 20 minute, 10ml air from the bottle was taken out by a injector and rapidly injected into a connector of the CY-3 type digital oxygen analyzer with the oxygen analyzer indicating oxygen content of the pumped in air. The data, when stable, is recorded as the residual oxygen value, then the oxygen consumption (20min, 10ml) = 21% - residual oxygen value (%). (21% oxygen in air).

### 2.3.4 Measurement of heart rate of the mice

All the mice are suspended of food without suspension of water for 12 hours one day prior to the last medication, and heart rate is measured 3 hours later. Heart rate (HR) is calculated via the electrocardiogram, statistics is then conducted, and differences are compared.

### 2.3.5 Measurement of the level of thyroid hormone of serum of the mouse

A mouse is suspended of food without suspension of water for 12 hours subsequent to the last medication, and blood is taken from its eye ball, put in centrifugation for 15 minutes at 3500 r/min, serum is pumped out for measurement of T₃, T₄, FT₃, and FT₄ by means of radioimmunoassay. For detailed operations, see the instruction manual.

### 2.3.6 Measurement of heart indexes of the mice

A mouse is sacrificed its heart instantly taken out for measurement of its heart weight (HW) and (left ventricular weight (LVW). Heart weight index (HWI=HW/BW) and left ventricular weight index (LVWI=LVW/BW) is calculated at conclusion the experiment.

### 2.4 Statistics

Results were expressed as mean ± standard deviation (*x̅*±s), SPSS 19.0 is adopted as the statistical software for statistical analysis. For multi-group comparison, one-way variance analysis is adopted, for group-group comparison, q test is adopted for variance with homogeneity, and Dunnett's T3 test is adopted for non-homogeneous variance. *p* < 0.05 are considered as significant.

### 3 Experiment results

### 3.1 Results for determination of success of the model

### 3.1.1 Effect on heart rate of a mouse

Compared with the blank group, the mice in the model group have a highly increased trend of heart rate (HR) at the 6th day of administration of L-Thyroxine Sodium salt solution (with significance P<0.001)).

(See Table 1)

**Table 1 Effect of 6 day injection of levothyroxine sodium (x̅±S)**

| group | Dosage (µg/kg) | number | HR (times/min) |
|---|---|---|---|
| blank | - | 10 | 470.46±105.30 |
| model | 350 | 10 | 674.42±57.61^{ΔΔΔ} |

| | | | |
|---|---|---|---|
| Remark: as compared with the blank group, ΔΔΔP<0.001 | | | |

### 3.1.2 Effect on T₃ and T₄ of serum of the mice

Compared with the blank group, levels of T₃ and T₄ in the model group are substantially increased (P<0.001). (See Table 2)

**Table 2 Effect of 7 day injection of levothyroxine sodium on T₃ and T₄ (x̅±S)**

| group | dosage (µg/kg) | number | T3(ng/ml) | T4(ng/ml) |
|---|---|---|---|---|
| blank | - | 10 | 0.36±0.08 | 29.47±7.63 |
| model | 350 | 10 | 2.89±0.97^{ΔΔΔ} | 311.43±50.76^{ΔΔΔ} |

| | | | | |
|---|---|---|---|---|
| Remark: as compared with the blank group, ΔΔΔP<0.001 | | | | |

### 3.1.3 Effect on heart weight index of a mouse

Compared with the blank group, subsequent to 7 days of medication, the heart weight index and the left ventricular weight index of the mice in the model group are significantly elevated (P<0.001). (See Table 3)

**Table 3 Effect of 7 day injection of levothyroxine sodium on the heart weight index of a mouse (x̅±S)**

| group | Dosage (µg/kg) | number | HWI (mg/g) | LVWI (mg/g) |
|---|---|---|---|---|
| blank | - | 10 | 4.473±0.202 | 2.911±0.263 |
| model | 350 | 10 | 5.642±0.295^{ΔΔΔ} | 3.735±0.378^{ΔΔΔ} |

| | | | | |
|---|---|---|---|---|
| Remark 1: as compared with the blank group, ΔΔΔP<0.001 Remark 2: HWI (hear weight index) =HW/BW, LVWI (left ventricular weight index) =LVW/BW | | | | |

In conclusion, the mice in the model group, subsequent to 7 days of medication, compared with the blank group, have significantly elevated heart rate, T3 and T4 in the serum and heart weight index (P<0.001). Thus the model is successful and medication is feasible.

### 3.2 Effect of catalpol on appearance and behavior of the mice in the model group

During the course of the experiment, the mice in the blank group show no abnormality with their activity, mental status, or color of hair. The mice in the hyperthyroidism group starts to show dysphoria and increased frequency of activities, are more excitable, and particularly so during subcutaneous injection. Further, food and water intake increase, the cage becomes damper, and amount of excrement increases. As compared with the blank group, the mice in the model group have drier and darker hair. The mice in the catalpol group are quieter and have reduced symptoms of dysphoria, excitability, and drier and darker hair as compared with the model group.

### 3.3 Effect of catalpol on the body weight of mice in the model group

There was no significance difference among groups in the pre-treatment body weight, the post-treatment body weight, and the difference therebetween of the mice., except that the mice in the model group increase less of their body weight comparing with the blank group. Compared with the model group, the mice in the catalpol group and in the methimazole group have more body weight increase to some extent. (See Table 4)

**Table 4 Effect of catalpol on the body weight of mice in the model group (x̅±S)**

| group | Dosage (mg/kg) | number | BW1(g) | BW2(g) | BW2-BW1 (g) |
|---|---|---|---|---|---|
| blank | - | 10 | 27.50±2.68 | 37.60±3.24 | 10.10±1.10 |
| model | - | 8 | 27.38±3.11 | 35.75±2.92 | 8.38±2.97 |
| Catalpol low dosage | 30 | 10 | 27.40±2.91 | 36.40±3.63 | 9.00±3.52 |
| Catalpol medium dosage | 60 | 10 | 27.70±3.50 | 37.80±5.03 | 10.10±2.38 |
| Catalpol high dosage | 90 | 10 | 27.60±2.88 | 37.75±3.03 | 10.15±2.43 |
| methimazole | 7.5 | 10 | 27.30±3.71 | 36.30±4.37 | 9.00±1.05 |

### 3.4 Effect of catalpol on oxygen consumption of mice in the model group

As compared with the blank group, the mice in the model group have significantly elevated oxygen consumption, the difference is significant (P<0.001). As compared with the model group, the mice in the catalpol low dosage group, the catalpol high dosage group and the methimazole group have significantly decreased oxygen consumption, the difference being statistically significant (P<0.001). (See Table 5)

**Table 5 Effect of catalpol on oxygen consumption of mice in the model group (x̅±S)**

| group | Dosage (mg/kg) | number | Decrease in oxygen content (%, 20min) |
|---|---|---|---|
| blank | - | 10 | 6.70±1.13 |
| model | - | 9 | 1 0.09±0.87^{ΔΔΔ} |
| Catalpol low dosage | 30.0 | 10 | 8.88±1.36^{▲} |
| Catalpol medium dosage | 60.0 | 10 | 9.03±1.53 |
| Catalpol high dosage | 90 | 10 | 8.81±1.42^{▲} |
| methimazole | 7.5 | 10 | 8.53±2.41^{▲} |

| | | | |
|---|---|---|---|
| Remark1: as compared with the blank group, ΔΔΔP<0.001; as compared with the model group, A P<0.05 | | | |

### 3.5 Effect of catalpol on the heart rate of mice in the model group

As compared with the blank group, the mice in the model group have significantly increased heart rate, where P<0.001. As compared with the model group, the mice in the catalpol groups and in the methimazole group have decreased heart rate to some extent, in particular, the difference of the mice in the catalpol medium dosage group and the catalpol high dosage group as compared with the model group is significant (P<0.001); the heart rate of the mice in the catalpol low dosage group and in the methimazole group is significantly decreased (P<0.001). (See Table 6)

**Table 6 Effect of catalpol on heart rate of mice in the model group (x̅±S)**

| group | Dosage (mg/kg) | number | Heart rate (times/min) |
|---|---|---|---|
| blank | - | 10 | 343.22±82.89 |
| model | - | 8 | 668.26±91.98^{ΔΔΔ} |
| Catalpol low dosage | 30 | 10 | 507.56±80.82^{▲▲▲} |
| Catalpol medium dosage | 60 | 10 | 538.75±118.69^{▲▲} |
| Catalpol high dosage | 90 | 10 | 529.63±114.20^{▲▲} |
| methimazole | 7.5 | 10 | 474.02±95.94^{▲▲▲} |

| | | | |
|---|---|---|---|
| Remark: as compared with the blank group, ΔΔΔP<0.001; as compared with the model group, ▲▲ P<0.01, ▲▲▲ P<0.001 | | | |

### 3.6 Effect of catalpol on T3 and T4 of mice in the model group

As compared with the blank group, the mice in the model group have significantly elevated level of T₃ in the serum, (P<0.001). As compared with the model group, the mice in the catalpol groups and in the methimazole group have decreased T₃ level in the serum to some extent: the difference of the mice in all the three catalpol groups and the methimazole group with the model group is significant (P<0.05). (See Table 7)

As compared with the blank group, the mice in the model group have significantly elevated level of T₄ in the serum, (P<0.001). As compared with the model group, the mice in the catalpol groups and in the methimazole group have decreased T₄ level in the serum to some extent, the difference of the mice in the methimazole group comparing with the model group is significant (P<0.05). (See Table 7)

**Table 7 Effect of catalpol on T3 and T4 of mice in the model group (x̅±S)**

| group | Dosage (mg/kg) | number | T3(ng/ml) | number | T4(ng/ml) |
|---|---|---|---|---|---|
| blank | - | 10 | 0.81±0.35 | 10 | 49.11±20.64 |
| model | - | 8 | 4.96±0.81^{ΔΔΔ} | 8 | 223.46±81.61^{ΔΔΔ} |
| Catalpol low dosage | 30 | 10 | 3.84±1.04^{▲} | 10 | 181.02±90.13 |
| Catalpol medium dosage | 60 | 10 | 3.52±1.26^{▲} | 9 | 179.90±50.24 |
| Catalpol high dosage | 90 | 10 | 3.50±1.35^{▲} | 10 | 178.50±52.40 |
| methimazole | 7.5 | 9 | 3.64±0.86^{▲} | 8 | 145.61±49.43^{▲} |

| | | | | | |
|---|---|---|---|---|---|
| Remark: as compared with the blank group, ΔΔΔP<0.001; as compared with the model group, ▲ P<0.05, ▲ ▲P<0.01 | | | | | |

### 3.7 Effect of catalpol on FT₃ and FT₄ of mice in the hyperthyroidism model group

As compared with the blank group, the mice in the model group have significantly elevated level of FT₃ in the serum, the difference being significant (P<0.001). As compared with the model group, the mice in the catalpol groups and in the methimazole group have decreased FT₃ level in the serum to some extent: compared with the model group, the difference of the mice in the catalpol low dosage group is significant (P<0.01); the difference of FT₃ index of the mice in the catalpol medium dosage group, the catalpol high dosage group and in the methimazole group, comparing with the model group, is also significant (P<0.05). (See Table 8)

Compared with the blank group, the mice in the model group have significantly elevated level of FT₄ in the serum (P<0.001). As compared with the model group, the mice in the catalpol groups and in the methimazole group have decreased FT₄ level in the serum to some extent, in particular, the difference of the mice in the catalpol low dosage group with the model group is significant (P<0.05). (See Table 8)

**Table 8 Effect of catalpol on FT3 and FT4 of mice in the hyperthyroidism model group (x̅±S)**

| group | Dosage (mg/kg) | number | FT₃(ng/ml) | number | FT₄(ng/ml) |
|---|---|---|---|---|---|
| blank | - | 10 | 1.10±0.35 | 10 | 6.58±2.22 |
| model | - | 8 | 11.40±2.49^{ΔΔΔ} | 8 | 32.49±10.51^{ΔΔΔ} |
| Catalpol low dosage | 30 | 10 | 7.49±2.40^{▲▲} | 10 | 22.87±5.12^{▲} |
| Catalpol medium dosage | 60 | 10 | 8.22±3.26^{▲} | 9 | 23.77±4.27 |
| Catalpol high dosage | 90 | 10 | 7.56±2.15^{▲} | 10 | 24.02±5.14 |
| methimazole | 7.5 | 9 | 8.80±2.31^{▲} | 8 | 26.85±7.78 |

| | | | | | |
|---|---|---|---|---|---|
| Remark: as compared with the blank group, ΔΔΔP<0.001; as compared with the model group, ▲▲ P<0.01, ▲P<0.05 | | | | | |

### 3.8 Effect of catalpol on heart weight index of mice in the model group

compared with the blank group, the mice in the model group have significantly elevated heart weight index, (P<0.001). compared with the model group, the mice in the medication groups and in the methimazole group have decreased heart weight index to some extent: the difference of heart weight index of the mice in the catalpol medium dosage group and the catalpol high dosage group is significant (P<0.001); the heart weight index of the mice in the methimazole group as compared with the model group is also significantly decreased (P<0.05). (See Table 9)

compared with the blank group, the mice in the model group have significantly elevated left ventricular weight, where P<0.001. compared with the model group, the mice in the three catalpol dosage groups and the methimazole group have decreased left ventricular index to some extent: the mice in the catalpol medium dosage group and the catalpol high dosage group have significant difference in left ventricular weight as compared with the model group (P<0.01). (See Table 9)

**Table 9 Effect of catalpol on heart weight index of mice in the model group (x̅±S)**

| group | Dosage (mg/kg) | number | HWI | LVWI |
|---|---|---|---|---|
| blank | - | 10 | 4.351±0.302 | 1.193±0.253 |
| model | - | 8 | 6.325±0.521^{ΔΔΔ} | 1.983±0.303^{ΔΔΔ} |
| Catalpol low dosage | 30 | 10 | 5.912±0.453 | 1.786±0.143 |
| Catalpol medium dosage | 60 | 10 | 5.650±0.293^{▲▲} | 1.625±0.234^{▲▲} |
| Catalpol high dosage | 90 | 10 | 5.58±0.547^{▲▲} | 1.582±0.466^{▲▲} |
| methimazole | 7.5 | 10 | 5.784±0.290^{▲} | 1,560±0.222^{▲▲} |

| | | | | |
|---|---|---|---|---|
| Remark 1: as compared with the blank group, ΔΔΔP<0.001; as compared with the model group, ▲▲P<0.01, ▲P<0.05 Remark 2: HWI (hear weight index) =HW/BW, LVWI (left ventricular weight index) =LVW/BW | | | | |

### 4 Conclusion

Catalpol has the effect for treatment of hyperthyroidism and hyperthyroidism related cardiopathy.

## Claims

1. Catalpol for use in the treatment of hyperthyroidism.

2. Catalpol for use in the treatment of hyperthyroidism related cardiomyopathy.

## Patentansprüche

1. Catalpol zur Behandlung von Hyperthyreose (Schilddrüsenüberfunktion).

2. Catalpol zur Behandlung von Hyperthyreose-bedingter Kardiomyopathie.

## Revendications

1. Catalpol pour une utilisation dans le traitement de l'hyperthyroïdie.

2. Catalpol pour une utilisation dans le traitement de la cardiomyopathie liée à l'hyperthyroïdie.
